# EUROPEAN PATENT APPLICATION

(11) **EP 4 781 953 A1**
(43) Date of publication of application: **29.07.2026**
(21) Application number: 25154393.0
(22) Date of filing: 28.01.2025
(51) Int. Cl.: A61F 5/058, A61F 5/32, A61H 1/02, A63B 23/03

(54) **MEDICAL DEVICE FOR PHYSICAL REHABILITATION OF TEMPORARY ANDIBULAR DISORDER (TMD)**

(71) Applicant: UAB Isences, 44313 Kaunas (LT)
(72) Inventor: Siudikas, Adakrius, 44313 Kaunas (LT)
(74) Representative: Metida

(57) **Abstract**

The invention provides the medical device which is intended for use in the physical rehabilitation in the case of temporomandibular disorders that limit normal and pain-free mouth opening, impaired chewing associated with dysfunction of the masticatory muscles, temporomandibular joints (TMJ) and related musculoskeletal structures. The medical device functions by simulating the natural movements of the temporomandibular joint through controlled pneumatic inflation. The device is designed to provide gentle, safe and precisely controlled movement of the TMJ and mandible. The medical device is a pneumatically adjustable rehabilitation device for temporomandibular disorder treatment, leveraging advanced bioengineering and 3D printing technology. The device is designed to support precise, controlled jaw movement, simulating natural temporomandibular joint (TMJ) kinematics, and aiding in physical rehabilitation through pneumatic expansion and contraction.

## Description

### FIELD OF THE INVENTION

The present invention relates to a new bioengineering concept medical device based on the functional biomechanics of the mandible and related structures and intended for the physical rehabilitation of temporomandibular disorder (TMD).

### BACKGROUND OF THE INVENTION

TMD is a collective term for a group of musculoskeletal conditions involving pain and/or dysfunction in the masticatory muscles, temporomandibular joints (TMJ) and associated structures and limiting normal and pain-free mouth opening. TMD are the second-most prevalent musculoskeletal disease that causes pain and disability affecting 5% - 12% of the population (G. Minervini 2022).

The operation of TMD physical rehabilitation medical device follows the principle of TMJ functional anatomy. Evaluating the principle of operation Physio Care would be most effectively applied to myogenic/functional disorders of masticatory muscles - spasm TMD. Myogenic TMD (M-TMD) - the most common 43.5% of all TMD cases, characterized by local or regional pain in the muscles of mastication, increased sensitivity, difficulty in chewing and limited opening of the mouth. The prevalence of M-TMD is difficult to accurately estimate, but the global prevalence is between 3% and 15% of the general population. It has been shown that there is no single and universally successful treatment management for M-TMD. According to a recent network meta-analysis, the most effective treatment methods for M-TMD are device-based and manual maxillofacial therapy or physical therapy ( EA Al, Moraissi 2020). The treatment of TMD's varies depending on the etiology and involves treatment methods and medical devices used by general practitioners, otolaryngologists, dentists, maxillofacial surgeons, orthognathic surgeons, and physical therapists. Scientific evidence proves that the combination of different treatment methods with physical therapy leads to the best results in the treatment of TMD (B. González-Sánchez 2023). Specialized physical rehabilitation therapy for TMD, combining manual therapy, exercise, physical procedures, and medical devices, has been shown to significantly improve quality of life ( Quintessence Int. 2018).

The functional elements of medical device of present invention are the basis of the new bioengineering concept and design medical device for TMD physical rehabilitation.

CN108309697(A) describes a temporomandibular joint traction apparatus and boosting device thereof. The boosting device comprises a pull-down component, a positioning component and an adjusting assembly. The pull-down component is provided with a hook for hooking lower gums, and the adjusting assembly is connected with the positioning component. The medical device of present invention allows to adjust the jaw opening pneumatically, not mechanically like a comparable analogue.

CN210144624(U) The device consists of an upper pressing plate and a lower pressing plate, the upper pressing head and the lower pressing parts are connected through a connecting piece and two airbags, which are connected to each other and connected to the inflation equipment through inflation pipes. The device can also be used for the maintenance of large instruments, used for the disassembly of precision parts, and can be used to make latex inflatable ball mouth openers and the like; and when the inflation equipment stops inflating, the expansion angle can be fixed. The medical device of present invention is a TMD rehabilitation device and enables rotational-sliding TMJ movement unlike an analog device that is mainly used for large instrument maintenance. US9220653 describes a therapeutic exercise device that can simultaneously increase the range of motion of the temporomandibular joint and strengthen the jaw muscles, comprising two bite elements with bite parts for insertion between the user's teeth and bellows between the bite elements that move them relative to each other towards an open position. Which separates the upper and lower jaws of the user. A spring applies a predetermined opening force to the opened bite parts, and one or more elastic elements attached between the bite elements apply an opposing closing force. A pneumatic pump controlled by the user supplies pressurized air to the bellows to open the bite elements when the closing force is sufficient to overcome the opening force. The device can be used with air bladders that massage the jaw muscles, with a choice of heat or cold. The device can be used with a mandibular translation adapter to exercise the user's mandibular posterior-anterior direction. The differences are that medical device of present invention focuses more on jaw opening and closing without advanced rotational/shear capabilities, whereas this invention supports bi-axial movement including rotational and shear movements, mimics physiological TMJ movement, provides separate air channels on the left and right sides allowing for asymmetrical adjustment of customized movements, is customized to individual anatomy using 3D printing and imaging data such as CT and MRI, and offers automated air adjustment systems for precise treatment protocols.

It was conducted interdisciplinary biomedical engineering experimental research by creating a temporomandibular joint (TMJ) /mandible model, combining the functional anatomy/biomechanics of the TMJ, materials science, and advanced 3D manufacturing technology.

The current state of art scientific approach offer several different methods of evaluating jaw muscles and condylar forces, depending on the degree of freedom of the jaw, the type of analysis and the set of surrounding orofacial tissue conditions. Evidence-based/modern biomechanical methods of the human jaw is experimental, using strain gauges, or innovative methods such as image correlation and numerical tests. Innovative numerical methods determine muscle activation profiles, condylar forces and stress strain fields in the human oral cavity are mainly based on joint degrees of freedom, but comparison of results remains a formidable task. With the added complexity of 3D printing technology, manufacturing material properties, the current finite element/experimental method is perhaps the most common tool used to study the specific problems of stress and strain characterization of the human jaw (M. Stefano 2024). Starting with the development of the TMD prototype concept, we set a complex task for the sustainability of the medical device: jaw biokinetic functionality, manufacturing technology, biocompatibility of materials, ergonomics, cost-effectiveness, and harmonious aesthetic design.

The new invention addresses the limitations of the prior art by incorporating advanced pneumatic regulation, independent control, biocompatibility, and diagnostic capabilities. It is a more comprehensive therapeutic approach to address broader TMD rehabilitation needs with innovative design features.

### SUMMARY OF THE INVENTION

The medical device operates by simulating the natural movements of the temporomandibular joint through controlled pneumatic inflation. It is designed to provide gentle, safe and precisely controlled movement of the TMJ and mandible.

The medical device is intended for the treatment of musculoskeletal disorders associated with pain in the muscles of mastication, temporomandibular joint (TMJ) and related structures, and/or temporomandibular disorder (TMD) that limit normal and pain-free mouth opening. TMD medical device would be most effectively applied to myogenic/ functional disorders of masticatory muscles - the most common 43.5% of all TMD cases. The rehabilitation of musculoskeletal structures is selected from the increasing the amplitude of movements of the temporomandibular joint and associated structures, strengthening the jaw muscles, restoring of the original muscle length and increasing of the dynamic strength of the masticatory muscles.

The medical device comprises a body, a front ergonomic/curved part which is designed to pick up/hold the device and place it in the oral cavity. Distal modelled body - for stable supporting contact of the upper jaw with the surfaces of the teeth when performing kinematic movements of the lower jaw.

At the bottom of the body/frame, symmetrically on both sides, there are two symmetrically positioned elastic inflatable functional elements, configured to perform kinematics/opening movements of the lower jaw.

The functional elements correspond to low-medium elastic hardness scores and this ensures physiologically safe functional adaptation in the oral cavity. The elastic and modelled upper contact surface of the body stably fits all upper/maxillary teeth: first, second, third - molars; first, second - premolars, canine; central, lateral - incisors. The elastic, inflatable functional element fits on both sides to the teeth of the lower jaw: first, second, third - molars; first, second - premolars, canine. The functional elements ensure controlled two-axis movement of the lower jaw, including rotational, translational, and shear directions.

Each of functional elements is inflatable-deflatable element and have separate air supply and exhaust channels that run inside the housing, and at the front part of it, they pass into flexible small-diameter tubes that are connected to the air supply/exhaust device by a connector. The air channels are positioned in each of the functional elements and configured to supply and exhaust air to functional elements, allowing controlled, adjustable movements of the mandible in three axes: rotation, translation and lateral deviation.

The functional element includes internal modulating membrane, which ensures stability and enables precise rotational-shear jaw movements during inflation and deflation.

The TMD body and functional elements can be proportionally small, medium, and large in size, suitable for children, teenagers, and adults, respectively. In the case of specific TMD, a personalized medical device is manufactured based on CT parameters of the oral cavity. When the air is exhausted from the functional elements, the TMD acquires a low profile and can be inserted into the oral cavity with a very restrictive opening of up to 1 cm. The functional element is activated, when air is supplied to it, and a symmetrical rotational and translational movement of the functional element with the lower jaw is generated. The dynamic quantitative change in the displacement of the functional element is proportional to the pressure of the supplied air. The total force direction vector of the functional element generated by the air pressure is directed to all the contact surfaces of the teeth of the lower jaw, which generates a physiological/levitating, protecting/unloading load on the temporomandibular joint, and at the same time ensuring the rotational/translational movement of the lower jaw. The air injection is not limited by the force generated by human muscles, air supply from an external source with pressure measurement can also be used.

The combination of the elastic properties of the material of the functional element, the external shape - bellows, without limiting their number, and the internal structures - the supporting/modulating membranes determines the generation of rotational and translational movement of the functional element.

When air of different pressure is supplied to the individual functional elements, rotational, translational and lateral deviation movement of the functional element is created as 3D dynamic jaw movement. The dynamic quantitative change in the displacement of the functional element is proportional to the pressure of the supplied air.

The medical device further comprises a pneumatic control system configured for: a) symmetric inflation of the functional elements to facilitate physiological balanced, two-axis mandible movements, and b) asymmetric inflation of the functional elements to generate precise, patient-specific three-dimensional mandible movements: rotation, translation and lateral deviation, wherein elasticity of the functional element respectively filled with air pressure ensures a uniform isotonic muscle tension.

The pneumatic control system includes air pressure regulation mechanism, manual or automated, configured to control the stretching intensity of the masticatory muscles, temporomandibular joints (TMJ) and related musculoskeletal structures.

The body of medical device includes an upper contact surface ergonomically designed to fit maxillary teeth; and a lower contact surface configured to distribute forces evenly on mandibular teeth. The body is constructed from 3D-printed, biocompatible elastic materials ensuring stability and patient comfort, wherein the biocompatible material and 3D-printed structure allow for patient-specific customization based on computed tomography (CT) or magnetic resonance imaging (MRI) data.

The medical device provides advanced diagnostic compatibility, including 4D motion tracking for real-time assessment of jaw kinematics.

### BRIEF DESCRIPTION OF DRAWINGS

Fig. 1 shows movements of temporomandibular joint.
Fig. 2 shows arthrokinematics of opening the mouth.
Fig. 3 shows normal functional movement of the condyle and disc during the full range of opening and closing.
FIG. 4 shows front/side perspective view of a device / single bellows.
Fig. 5 shows front/side perspective view of a device / double bellows.
Fig. 6 shows right side view of the device / sinle bellows.
Fig. 7 shows right side view of the device / double bellows.
Fig. 8 shows rear/side perspective view of the device.
Fig. 9 shows rear view of the device.
Fig. 10 shows view from the rear bottom rotated device.
Fig. 11 shows left side view of the device.
Fig. 12 shows front view of the device / single bellows.
Fig. 13 shows front view of the device / double belows.
Fig. 14 shows a top view of the device.
Fig. 15 shows a bottom view of the device.
Fig. 16 shows front-side cross-sectional view of the functional element of the device.
Fig. 17 shows a rear view of the middle section of the functional element of the device.
Fig. 18 shows side view of the device after suctioning air.
Fig. 19 shows side view of the device after inflation.
Fig. 20 shows front/side perspective view of a device / transparent internal structures.
Fig. 21 shows right side view of the device / transparent internal structures.
Fig. 22 shows front view of the device of the invention / transparent internal structures.
Fig. 23 shows rear view of the device / transparent internal structures.
Fig. 24 shows a bottom view of the device / transparent internal structures.
Fig. 25 shows view of the device before placement in the oral cavity.
Fig. 26 shows deflated device inserted into the oral cavity.
Fig. 27 shows an inflated device in the oral cavity.
Fig. 28 shows TMD Physio care medical device.
Fig. 29 shows deflated TMD Physio care device.
Fig. 30 shows inflated TMD Physio care device.

### DETAILED DESCRIPTION OF THE INVENTION

The medical device is a pneumatically adjustable rehabilitation device for temporomandibular disorder treatment, leveraging advanced bioengineering and 3D printing technology. The medical device is designed to support precise, controlled jaw movement, simulating natural temporomandibular joint (TMJ) kinematics, and aiding in physical rehabilitation through pneumatic expansion and contraction.

The medical device's core design elements and mechanisms are outlined in the drawings, providing a comprehensive understanding of its structure, functionality, and operational principles.

**Two-Axis Movement.** The medical device generates rotational and shear movement of the lower jaw, mimicking natural TMJ motion. The functional element of medical device is characterized by the generated two-axis movement. The medical device generates rotational and shear movement of the lower jaw, mimicking natural TMJ motion. This ensures a physiological movement of the TMJ, with a protective/sparing effect on the biomechanical load of the temporomandibular joint when the masticatory muscles are relaxed during the stretching exercise.

**Safe & Gentle Jaw Mobilization:** The air-assisted mechanism ensures that forces are applied in a controlled manner, minimizing the risk of injury.

The invention surpasses existing solutions by offering a pneumatically controlled stretching function, which is more precise and adaptable compared to conventional mechanical jaw rehabilitation devices.

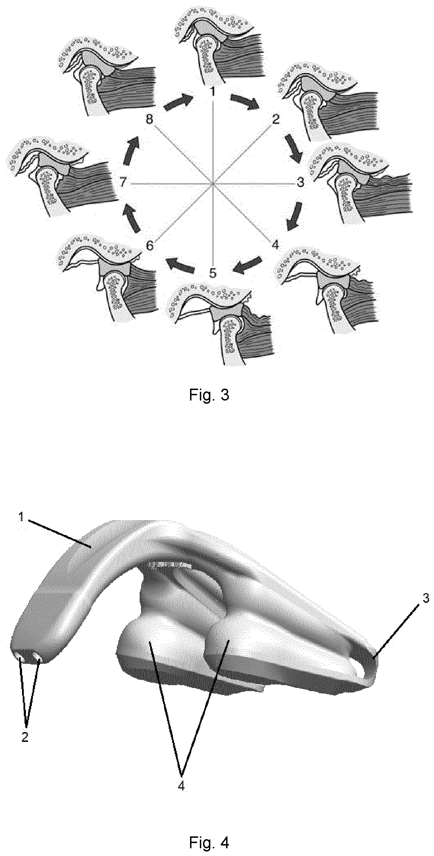

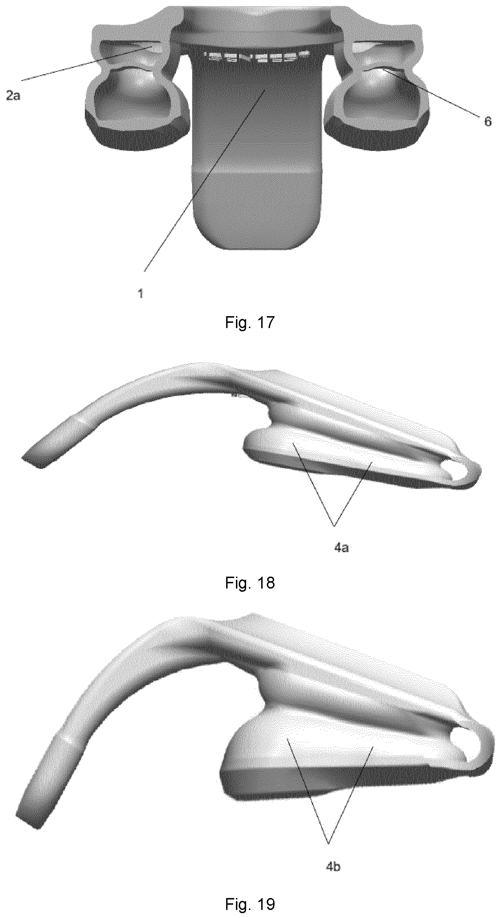

**Functional Elements.** The medical device incorporates two integrated functional elements, either single or double bellows, as illustrated in Figures 4 - 7, 12 - 13. Air pressure is applied to the functional elements, causing them to expand or contract and thereby influencing the movement of the lower jaw (Figures 18 - 19, 29 -30). When inflated, the functional element generates a controlled forward and/or lateral protrusion of the jaw. When functional element is deflated, the jaw naturally retracts to its resting position. The operation of the functional elements enables a rotation-shear movement of the lower jaw, which mimics physiological TMJ function. This provides a protective and sparing effect on the TMJ, reducing excessive biomechanical loading while promoting relaxation of the masticatory muscles.

**Independent Control of Left and Right TMJ.** Air is supplied through two separate air channels, enabling symmetric or asymmetric movement adjustments. The movement of the lower jaw is generated by two functional elements integrated into the body and acting simultaneously on both left and right sides TMJ. Air is supplied to the functional elements through two separate air channels, one foreach functional element, located in the housing. When the same pressure is applied to both functional elements, the two-axis movement of the lower jaw is modelled. By applying different pressure to a separate functional element, asymmetric combined movements of the lower jaw are modelled in 3D dimensions.

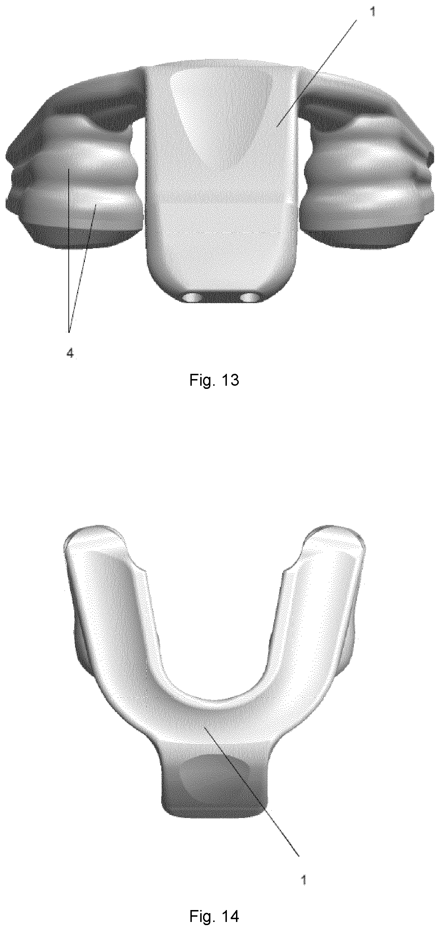

**Pneumatic Control System.** The medical device comprises two independent air channels, one for each TMJ (Figures 14, 16-17). These air channels supply air to the bellows, allowing for controlled, adjustable movement of the jaw. The air channels ensure symmetric and asymmetric inflation capabilities. Symmetric inflation applies the same pressure to both sides, ensuring a balanced, two-axis movement of the jaw. Asymmetric inflation applies varied pressure to either side, enabling asymmetrical stretching and customized adjustments for patient-specific therapy.

The Air Pressure Regulation Mechanism provides air pressure which can be manually adjusted using an external pump or automated system. This allows precision dosing of the stretching intensity, ensuring gentle and safe mobilization of the TMJ without excessive force. Pneumatically (with the help of the hand) adjustable air inflation enables gentle, safe and purposefully dosed movement-protrusion of the lower jaw. **Precision-Controlled Inflation & Deflation Mechanism.** The user or therapist can manually adjust the air pressure levels based on the required jaw movement and stretching intensity. Optional manual or automated air regulation can be implemented for programmed treatment protocols. Once air is released, the device gradually deflates, allowing the jaw to return to its natural resting position. This prevents excessive stress on the TMJ and ensures a smooth recovery phase between exercises.

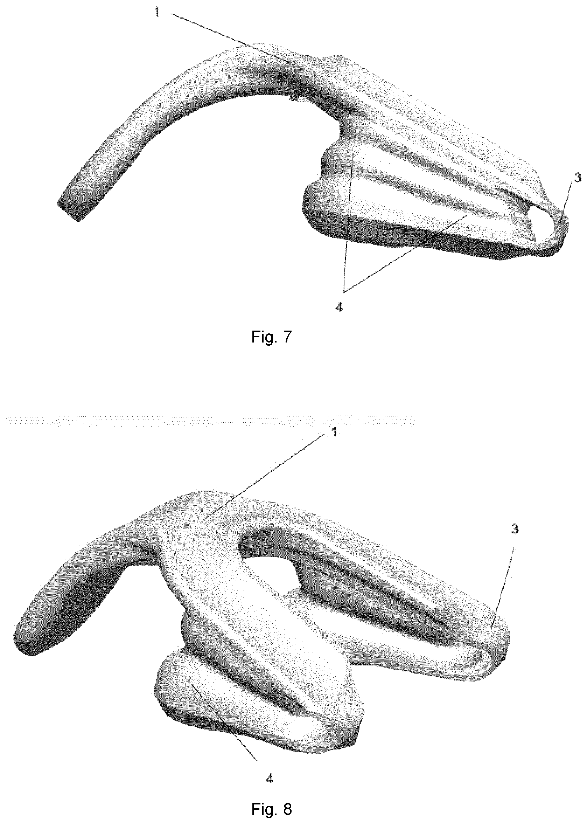

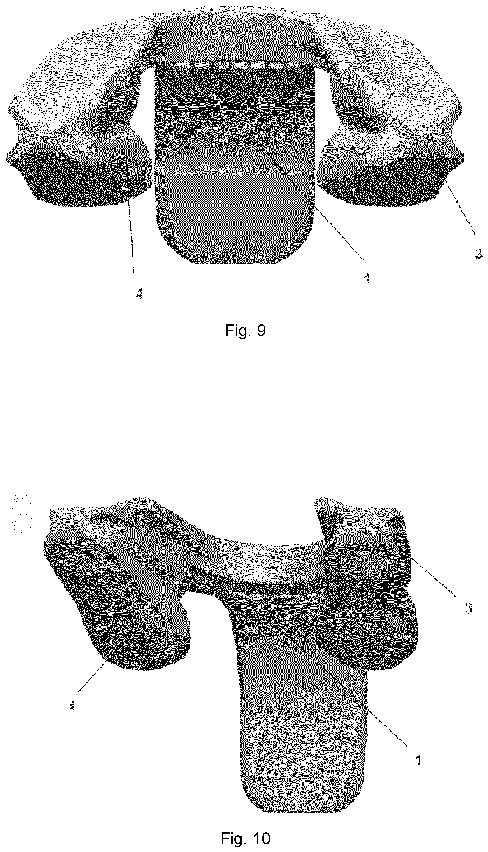

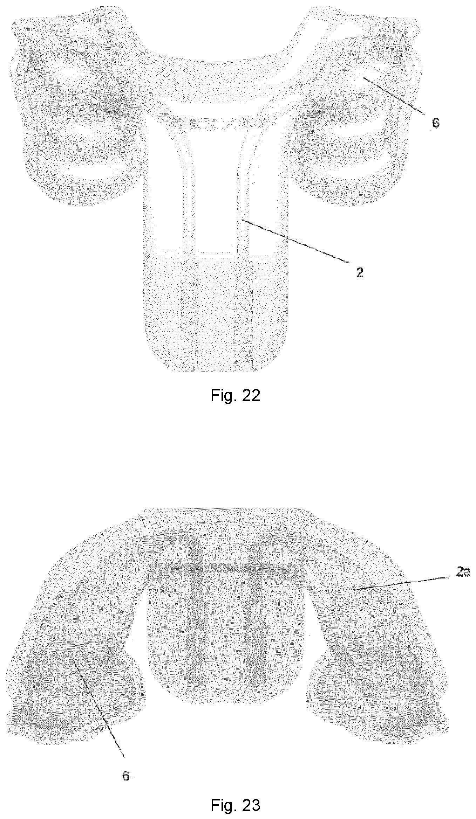

**3D-Printed Biocompatible Structure and Flexible and Adaptive Design.** The medical device is manufactured using biocompatible, elastic materials that conform to the user's dental and jaw structure (Figures 8-11, 20-27). This ensures stable contact adhesion with the teeth, preventing slippage and distributing force evenly. Designed for optimal comfort and usability, the medical device provides a non-invasive treatment approach. The material properties allow for safe and prolonged use in rehabilitation sessions. Biocompatible Elastic Material ensures stable adhesion to the teeth and even distribution of forces. The medical device is made of an elastic, biocompatible material that provides a stable contact adhesion with the surface of the teeth and evenly distributes the functional load to the upper and lower jaw.

**Advanced Imaging & Digital Diagnostics Compatibility.** The device supports 4D motion tracking of mandibular kinematics for precise real-time assessment. This allows healthcare providers to monitor rehabilitation progress and adjust treatment accordingly. The device can be used in conjunction with CT scans and MRI imaging to assess jaw movement and TMJ function. The design and fabrication process allows for patient-specific customization, ensuring a perfect anatomical fit. 3D printing technology enables rapid prototyping and modular device upgrades.

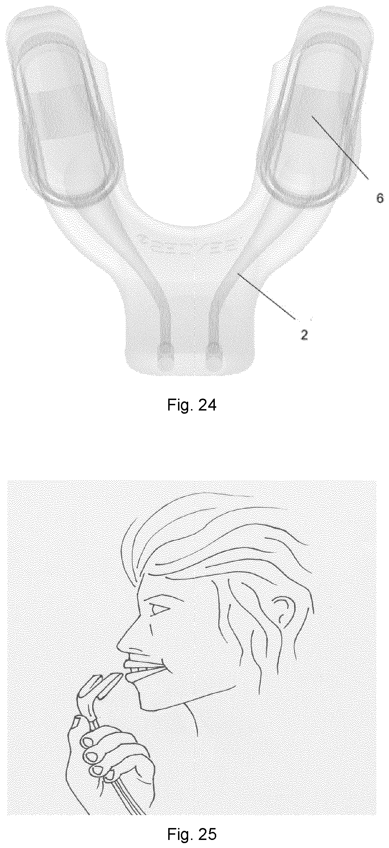

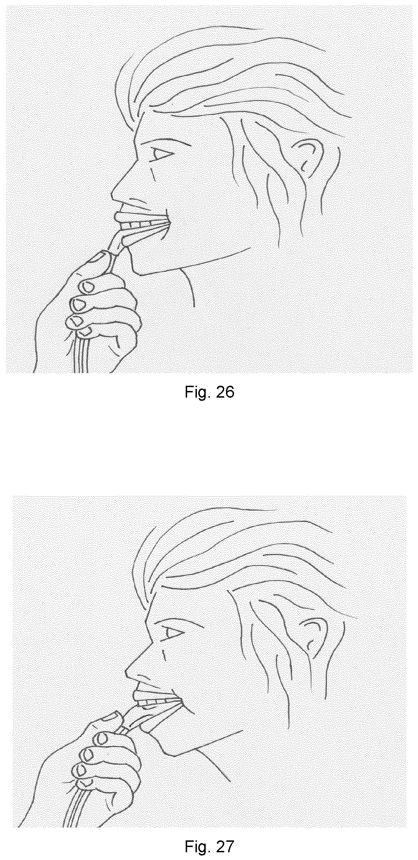

**Operational Mechanism and Use.** The medical device operates through a step-by-step functional process, as illustrated in Figures 25-27. The deflated device is positioned inside the oral cavity, fitting snugly between the upper and lower teeth (Figure 25). Air is pumped into the bellows, gradually expanding the device and gently stretching the jaw (Figures 26-27). This movement can be adjusted symmetrically or asymmetrically, depending on the rehabilitation needs. The patient or therapist adjusts the air pressure to achieve the desired level of protrusion, rotation, or lateral movement. The device allows for customized exercise programs, focusing on jaw muscle relaxation and TMJ decompression. The air is gradually released, allowing the device to deflate and the jaw to return to its natural resting position. This ensures safe muscle recovery without excessive strain. The process can be repeated based on the patient's treatment plan. The device offers variable air pressure settings, allowing for a progressive increase in jaw mobility over multiple sessions.

The current medical device innovation maturity level has been achieved through consistent applied research and experimental investigation set tests. The purpose of the investigation was to analyse the fundamental human functional anatomy of the TMJ in a 3D e-atlas and a detailed study of the temporomandibular joint (TMJ) biomechanics in order to gain new knowledge for specific jaw 2D kinematics. The new knowledge provided the insight to develop an experimental 2D kinematic model of the TMJ / jaw. An experimental testing model has been developed that is characterized by physiological: rotational/sliding movement of the TMJ element of mandibular movement. The finite element/experimental model evaluated the shear stress characteristics of biomedical grade set materials using impact force vector analysis and for the first time, dynamic internal pressure analysis of the prototype was performed on the model. The experimental set tests provided the knowledge to develop a new/original TMD prototype with physiological (2D) TMJ kinematic function.

Simultaneously, using forced vector analysis and computer-aided design (CAD) Design Software, TMD prototypes were designed and then test sets of 3D printed prototypes. The installation of an industrial-grade 3D printer on site made experimental modelling even more efficient. Additive manufacturing (AM) or three-dimensional (3D) printing is ideal /state-of-art for prototyping has introduced a novel production method in design, manufacturing, and distribution to end-users (Vosooghia A. 2019). Applying the most advanced 3D prototyping/manufacturing technology, sophisticated bioengineering, processing know-how, the TMD medical device V.1 model, CE certified, has been developed. The three-dimensional (3D) finite-element TMJ movement applied research work has provided new practical knowledge in creating accurate three-dimensional (3D) TMJ movement. In other words, to provide the TMD rehabilitation medical device with six degrees of freedom of jaw opening and closing movement. Precise three-dimensional (3D) movement of the TMJ is essential for interaction with three-dimensional, rotational, and translational movements. This new information, which provides knowledge of the 3D trajectory of mandibular movement, is essential for the comprehensive clinical application of the TMD rehabilitation management process. State-of-the-art 4D real-time 4D technology for mandibular kinematic assessment/TMJ analysis tool combined with CT and magnetic resonance imaging (MRI) morphological assessment of the orofacial region are indispensable tools for TMD diagnosis and appropriate treatment. A finite element/experimental model is compatible with input digital format and downstream (CAD)/computer aided manufacturing (CAM) process. The progressive integration of comprehensive digital diagnostics into medical device design and precise 3D manufacturing technology enables the creation of a customized TMD medical device for a detailed and consistent TMD physical rehabilitation protocol. The medical device aims for a breakthrough in TMD physical rehabilitation innovation by applying new concept bioengineering design and applying advanced 3D SLA manufacturing technology.

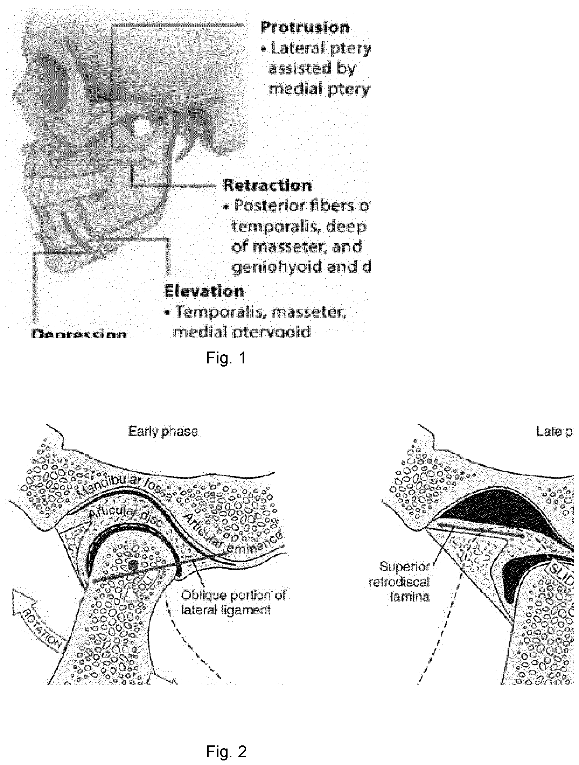

**Temporomandibular joint functional anatomy** - **basis of the medical device operation.** The medical device uses an active action, during which jaw opening tenses and relaxes the masticatory muscles and facial tissues, mobilizes the jaw and mouth, and as a result improves the function of the lower jaw. This medical device fundamentally differs from all known TMD medical devices in its effect on the temporomandibular joint (TMJ). There is a completely new biomedical engineering concept/design for medical device performance by functionally improving mandibular kinematics while preserving TMJ loading (decompressive levitation) during rotational/sliding movement of the temporomandibular joint (TMJ) during mouth opening. TMJ is the most functional joint and frequently used joint in our body. It is used during eating, speaking, yawning, kissing and sucking movements possible through this complex: depression - open mouth; elevation - closed mouth; lateral deviation (left and right) - side to side movement in chewing or grinding, retrusion - retraction of chin; protrusion - protraction of chin and various combinations of these movements (Figure 1).

The TMJ is a bi-articular joint that allows for complex movements. The TMJ is located between the head of the mandible and the mandibular fossa of the temporal bone. This TMJ complex consists of the TMJ, teeth, and soft tissues. The TMJ is a synovial, condylar, and hinge-like joint with fibrocartilaginous surfaces and an articular disc; this disc completely divides each joint into two cavities. In the upper TMJ cavity there is gliding, displacement or gliding, while in the lower cavity there is rotation or hinge movement (Figure 2).

**Jaw kinematics.** Functionally, mandibular movements are complex, with six degrees of possible motion occurring as complex interrelated rotational and translational activities. They are made possible by four different connections: lower and upper. Although the TMJ does not function independently of the other, it is necessary to classify the isolated movements of the lower jaw.

**Types of movements in three dimensions.** Condylar rotation and translation of the condyle-disc assembly, in most cases, begin simultaneously. On average, condylar rotation increases or decreases linearly by approximately2°/mm of anterior or posterior translation during opening or closing, respectively. Rotation occurs when the condyles rotate around a fixed point or axis during mandibular opening and closing.

**Rotational motion** can occur in three reference planes: horizontal, vertical, and sagittal. Each of them occurs around a point called the axis. Horizontal orientation axis: opening and closing movement, referred to as a hinge, therefore it occurs around an axis called the hinge axis. It is considered the purest rotation movement. Vertical axis of rotation: It occurs when oneof the condyles moves anteriorly from the position of the terminal hinge axis with the vertical axis in the opposite condyle, which remains in said axis. Sagittal axis of rotation: Occurs when one of the condyles moves inferiorly while the other remains in the position of the terminal axis. This movement occurs in conjunction with other movements. The amount of condylar rotation does not differ between men and women. A finding that contrasts with the greater maximum interincisal opening of men compared to women due to differences in jaw length. In fact, with the same degree of rotation, the greater the length of the mandible, the greater the opening of the mouth. Consequently, the degree of interincisal opening cannot be considered as a measure of joint mobility or laxity, unless corrected for mandibular size.

**Translational motion** can be defined as a movement in which every point of the object simultaneously has the same speed and direction. In the masticatory system, it occurs when the mandible protrudes. During normal movements, rotation and translation occur simultaneously, as the mandible rotates in one or more axes, each of the axes is changing orientation in space. The total movement of the mandible does not consist only of rotation and translation. Side-to-side or eccentric bodily movement of the mandible and rotation and translation of the joints indicate that the mandible acts as a free-moving or floating, structure. Controlled by pairs of complementary and opposing functional muscle groups that gradually exert impulse force with numerous force vectors, the three-dimensional movement of the mandible with a dual-operation joint system is unlike any other orthopaedic system in the body. Classical mandibular movements in terms of their geometry, using mechanical systems, in all three planes recoded in the Posselt diagram (Figure 3).

**Loads at the TMJ.** The efforts produced by the loads will generate a deformation which can be quantified by determining the change between the original length with the final length of a structure, this deformation is expressed as a percentage, there are twotypes of deformation: elastic one in which eliminating the force the material recovers its original dimension, while plastic deformation is one in which the original dimension is not recovered. The elastic limit es the yield point beyond which permanent deformation occurs, and the tissuedoes not return to its original shape.

It is recommended to start using the TMD Physio Care device after a manual, anatomical-functional clinical assessment of the TMJ and related structures by a specialized physiotherapist. The first step is to assess the possible gap between the incisors. If the gap is less than 15 - 25 mm, the device is deflated and, by grasping the curved part of the body, it is introduced into the oral cavity. Physio Care device is inserted into the mouth to the temporomandibular joint (TMJ). This is the location of the joint/system between the head of the lower jaw and the mandibular fossa of the temporal bone. This system consists of the TMJ, teeth and soft tissues. When the TMD device is properly localized, the functional element is inflated. At this time, the upper part of the body fits smoothly and stably against the surface of the upper jaw teeth, and the lower part of the functional element, contacting the surfaces of the lower jaw teeth, simulates the physiological - rotational / sliding movement of the lower jaw. Inflation of the functional element is continued until: 1. Clinical sign/symptom - without experiencing increased discomfort; 2. Until effective mouth opening is achieved - a sequence of rehabilitation exercises selected by a physiotherapist. Relaxation/stretching exercises are recommended to be performed on average 3-4 times a day. At that time, you should try to open your mouth as wide as possible until you feel a gentle stretch but not pain. The stretched/inflated functional element should be held for about 10 seconds, then deflate, relax and repeat all this 5-8 times. If the initial mouth opening is 25 mm or more, the TMD functional element does not need to be deflated, and the device can be directly inserted into the required position in the mouth. Next, relaxation/stretching exercises are performed, as in the sequence described above. All exercises usually consist of repeated attempts to open the mouth against applied resistance, usually divided into several sessions per day and continued for 2-3 months until optimal clinical results are achieved. In the later period, maintenance, lower intensity exercises may be used to ensure long-term physiologically complete mouth opening results.

Another advantage of the present invention is that the TMD medical device improves/restores chewing movements.

Another advantage of the present invention is that the medical device, by increasing the range of motion of the temporomandibular joint, strengthens the jaw muscles. Dynamic isotonic exercises for the masticatory muscles are performed by applying a counterforce to the movement being performed. For this purpose, the functional element is filled with a fixed amount of air, creating a fixed pressure in the functional element and repetitive chewing exercises of the masticatory muscles are performed. In individual cases - with asymmetrical needs for exercises to strengthen the masticatory muscles - different pressures can be created in the functional elements by air supplied through separate channels. Due to the elasticity of the functional element, the function of uniform muscle tension is ensured, which restores the original muscle length and increases the dynamic strength of the masticatory muscles.

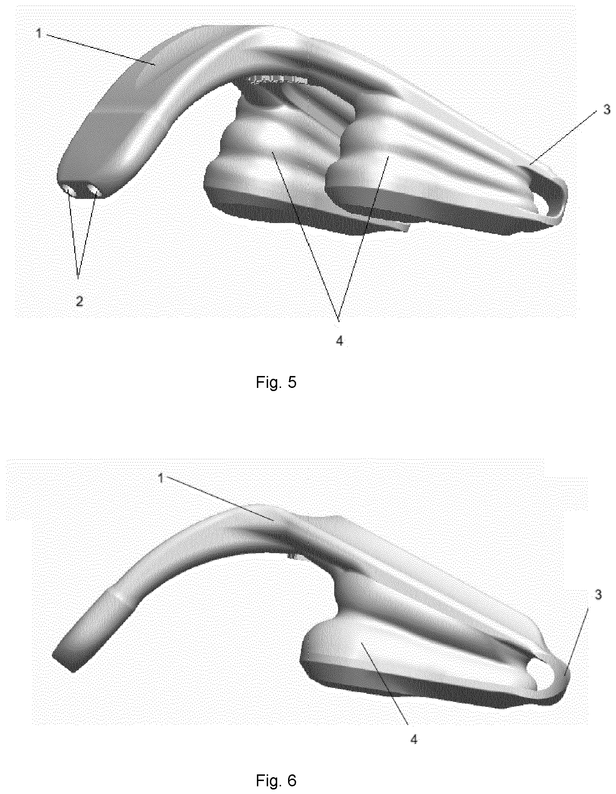

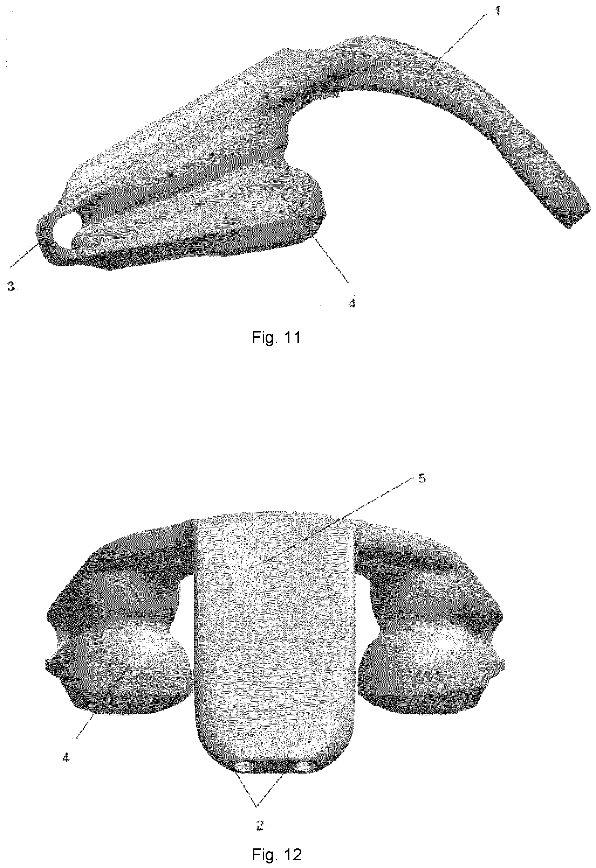

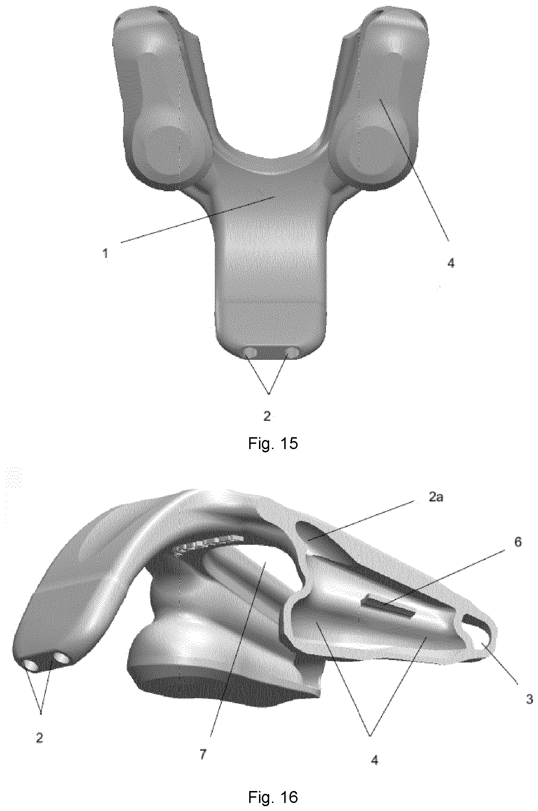

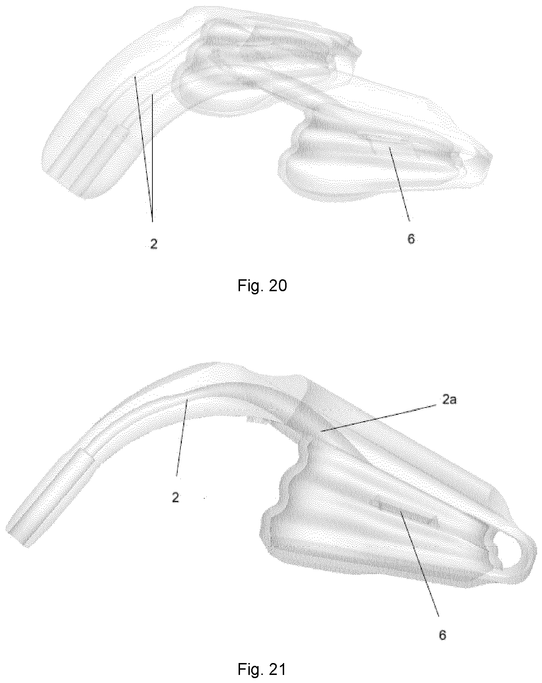

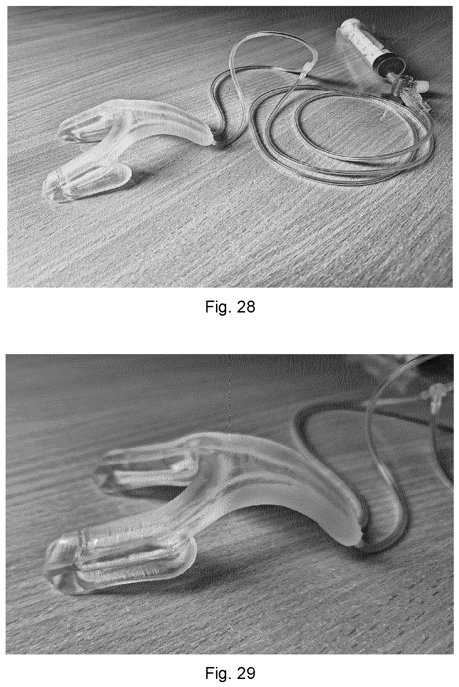

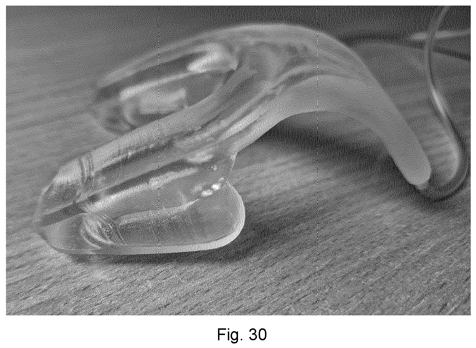

Referring to Fig. 4-17 TMD device comprises a body (1), which transitions to functional element (4) on both sides, air channels (2) and fitting support (3). The body part with functional elements is intended for the use of TMD physical rehabilitation exercises by introducing the device into the oral cavity (Fig. 25-27). The breathing space (7) between the body from above and between the functional elements from the front does not limit air breathing through the mouth (Fig. 16). The part of the body that is taken/held with the hand fingers is the ergonomic handle recess (5) of the body (Fig. 12) and air is supplied and extracted through the air channels (2) inside (Fig. 4; 5; 12; 15; 16) and internal structures, such as air channel inlets (2a) and modelling membrane (6) (Fig. 20; 21; 22). The functional element (4) (Fig. 4; 5; 6; 7; 8; 9; 10; 11; 12; 13; 14; 15) during air inflation ensure composite (two-axis): rotational-shear movement of the lower jaw is composed of an unlimited number of inflatable bellows. Demonstration of one bellows (Fig. 4; 6; 8; 9; 10; 11; 12) and two bellows (Fig. 5; 7; 13) TMD medical devices. The internal modulating membrane (6) (Fig. 16; 17; 20; 21; 22; 23) of the functional element (4) passes from the inner surface of the bellows and in complex interaction ensures rotational-shear movement of the lower jaw. Before use, the medical device is in standby position - neutral/ ambient atmospheric pressure (Fig. 4; 5; 28). Before placing the device in the oral cavity, air is aspirated, which ensures the placement of the TMD medical device in a limited mouth opening (Fig. 27; 29). Demonstrating the inflated functional element (Fig. 30) shows the functional state of TMD medical device, which ensures composite (two axis): rotational-shear movement of the lower jaw.

The TMD medical device is a cutting-edge, pneumatically controlled rehabilitation device for TMD treatment. By combining bioengineering principles, advanced material science, and digital diagnostics, it offers a customizable and patient-friendly solution that significantly improves TMJ rehabilitation outcomes. The modular, 3D-printed design, along with its precise inflation/deflation control system, makes it a superior alternative to existing TMD treatment devices.

## Claims

1. The medical device for temporomandibular disorder (TMD) physical rehabilitation, comprising:
a) a body designed for insertion into oral cavity,
b) two symmetrically positioned inflatable functional elements that ensure controlled two-axis movement of the lower jaw, including rotational, translational, and shear directions,
c) separate pneumatic air channels configured to supply and exhaust air from functional elements, allowing controlled, adjustable movements of the mandible in three axes:
rotation, translation and lateral deviation.

2. The medical device according to claim 1, wherein the functional element is inflatable-deflatable functional element.

3. The medical device according to any of claim 1 or 2, wherein the functional elements include internal modulating membranes, ensuring stability and enabling precise rotational-shear jaw movements during inflation and deflation.

4. The medical device according to any of claim 1-3, wherein the medical device is configured to reduce to a low-profile by deflating the air from the functional elements, allowing insertion into oral cavities with limited openings of up to 1 cm.

5. The medical device according to any of claims 1-4, further comprising a pneumatic control system configured for:
a) symmetric inflation of the functional elements to facilitate physiological balanced, two-axis mandible movements, and
b) asymmetric inflation of the functional elements to generate precise, patient-specific three-dimensional mandible movements: rotation, translation and lateral deviation,
wherein elasticity of the functional element respectively filled with air pressure ensures a uniform isotonic muscle tension.

6. The medical device according to any of claims 1-5, wherein the pneumatic control system includes air pressure regulation mechanism configured to control the stretching intensity of the masticatory muscles, temporomandibular joints (TMJ) and related musculoskeletal structures.

7. The medical device according to claim 6, wherein the air pressure regulation mechanism is selected from manual or automated air pressure regulation mechanism.

8. The medical device according to any of claims 1-7, wherein the body includes:
a) an upper contact surface ergonomically designed to fit maxillary teeth;
b) a lower contact surface configured to distribute forces evenly on mandibular teeth;
wherein the body is constructed from 3D-printed, biocompatible elastic materials ensuring stability and patient comfort.

9. The medical device according to any of claims 1-8, configured to provide 4D motion tracking for real-time assessment of jaw kinematics.

10. The medical device according to any of claims 1-9, wherein the biocompatible material and 3D-printed structure allow for patient-specific customization based on computed tomography (CT) or magnetic resonance imaging (MRI) data.

11. The medical device according to any one of claims 1-10, for use in the physical rehabilitation of temporomandibular disorders that limit normal and pain-free mouth opening, impaired chewing associated with dysfunction of the masticatory muscles, temporomandibular joints (TMJ) and related musculoskeletal structures.

12. The medical device for use according to claim 11, wherein the rehabilitation of musculoskeletal structures is selected from the increasing the amplitude of movements of the temporomandibular joint and associated structures, strengthening the jaw muscles, restoring of the original muscle length and increasing of the dynamic strength of the masticatory muscles.
